# EUROPÄISCHE PATENTANMELDUNG

(11) **EP 0 580 176 A1**
(43) Veröffentlichungstag der Anmeldung: **26.01.1994**
(21) Anmeldenummer: 93111885.5
(22) Anmeldetag: 23.07.1993
(51) Int. Cl.: A61L 2/10, A23L 3/28, A61L 2/00

(54) **Entkeimungsvorrichtung**

(30) Priorität: 23.07.1992 DE 4224308
(71) Anmelder: IMAB-STIFTUNG, FL-9496 Balzers (LI)
(72) Erfinder: Brück, Gernot K., Prof. Dipl.-Phys., D-50858 Köln (DE)
(74) Vertreter: Adler, Peter

(57) **Zusammenfassung**

Zur keimfreien Aufbewahrung bzw. zur Entkeimung von Lebensmitteln, medizinischen Geräten und dergleichen wird ein innen allseits verspiegelter Behälter vorgeschlagen, in dem eine UV-C-Licht aussendende Strahlenquelle (6) angeordnet ist. Der Behälter ist in geschlossenem Zustand völlig lichtdicht und eine Sicherheitselektronik gewährleistet, daß beim Öffnen des Behälters die UV-Lampe (6) ausgeschaltet wird. Um Lebensmittel vor Feuchtigkeitsverlusten zu schützen, wird eine Folie verwendet, die aus Fluorpolymer besteht.

## Beschreibung

Die Erfindung betrifft eine Vorrichtung zur Entkeimung und keimfreien Aufbewahrung von keimgefährdeten Gegenständen wie Lebensmitteln, Medikamenten, medizinischen Geräten und dergleichen.

Darüber hinaus betrifft die Erfindung eine Folie zum Abdecken oder Umhüllen von keimgefährdeten Gegenständen, insbesondere Lebensmitteln, zur Verwednung in einer gattungsgemäßen Vorrichtung.

Durch Mikroorganismen wie Bakterien oder Schimmelpilzen können Lebensmittel je nach Temperatur und Umgebung innerhalb von wenigen Stunden verderben.

Um die rasche Vermehrung dieser Mikroorganismen zu verhindern bzw. um Lebensmittel haltbar zu machen, werden insbesondere Kühlvorrichtungen verwendet, in denen eine gegenüber der Umgebung abgesenkte Temperatur erzeugt wird. Eine zweite häufig angewendete Möglichkeit zur Haltbarmachung von Lebensmitteln besteht in der chemischen Konservierung, der Evakuierung der Aufbewahrungsbehälter oder, insbesondere bei industrieller Anwendung, die Bestrahlung von Lebensmitteln. Hierbei wird eine UV-C-Strahlung, insbesondere die UV-C-Strahlung mit einer Wellenlänge von 253 nm verwendet, die eine stark keimtötende Wirkung hat.

Die Anwendung einer Bestrahlung von Lebensmitteln mit UV-C-Strahlung im großen Umfang scheiterte jedoch bisher u.a. an den umfangreichen Sicherheitsvorkehrungen, die für derartige Anlagen zu treffen sind.

Bei den zuvor beschriebenen Konservierungsverfahren kommt es häufig zu einer geschmacklichen Veränderung der Lebensmittel. Dies gilt insbesondere bei der Konservierung mit chemischen Stoffen, aber auch bei der Kühlung oder Tiefkühlung, wobei letztere z.B. bei bestimmten Gemüsesorten gar nicht angewendet werden kann.

Der Erfindung liegt die Aufgabe zugrunde, eine Entkeimungsvorrichtung zu schaffen, die es mit minimalem technischem Aufwand ermöglicht, Lebensmittel ohne geschmackliche Veränderung vor dem Verderben zu schützen bzw. andere keimgefährdete Gegenstände zu sterilisieren bzw. keimfrei aufzubewahren.

Diese Aufgabe wird gelöst durch einen lichtdicht verschließbaren Behälter mit einer UV-C-Licht-aussendenden Strahlenquelle, wobei der Behälter eine allseitige, die UV-C-Strahlung reflektierende Innenverspiegelung aufweist.

Die erfindungsgemäße Vorrichtung besteht aus einem lichtdichten Behälter, der z.B. mit einer 9 W Niederdruck-Quecksilberdampflampe ausgerüstet ist.

Der Behälter ist auf seiner Innenwandung allseits verspiegelt, so daß die UVC-Strahlung nahezu vollständig reflektiert wird und keine gegen die Strahlung abgeschatteten Bereiche im Inneren des Behälters bestehen.

Um die keimgefährdeten Gegenstände auch von ihrer Unterseite bestrahlen zu können, kann der Behälter eine im Abstand zum Behälterboden angeordnete UV-lichtdurchlässige Auflage aufweisen, die beispielsweise aus Quarzglas oder einem Fluorpolymerkunststoff bestehen kann.

Die Auflage kann selbstverständlich auch als Schale ausgebildet sein, in der z.B. flüssige Lebensmittel aufbewahrt werden können. Die Auflage ist zweckmäßigerweise auf Abstandshaltern auf dem Behälterboden aufgeständert, wobei diese Abstandshalter zur Vermeidung von Lichtschattengebieten ebenfalls aus UV-lichtdurchlässigem Material bestehen können.

Um sicher zu verhindern, daß die UV-Strahlenquelle eingeschaltet ist, wenn der Behälter geöffnet wird, kann er eine Sicherheitsschaltung aufweisen, die die UV-Lampe beim Öffnen abschaltet. Die Sicherheitsschaltung kann z.B. aus mindestens zwei in Reihe geschalteten Mikroschaltern bestehen, die im Bereich der Öffnungsklappe angeordnet sind und beim Öffnen unmittelbar ein Ausschalten bewirken.

Da das Innere des Behälters nach verhältnismäßig kurzer Zeit durch die erzeugte UV-Strahlung keimfrei ist, muß die UV-Strahlenquelle nicht ständig in Betrieb sein. Aus diesem Grunde kann eine zweckmäßige Zeitschaltelektronik vorgesehen sein, die die Leuchtdauer der Strahlenquelle begrenzt oder die die Strahlenquelle intervallmäßig ein- und ausschaltet.

Die Zeitschaltelektronik kann dabei so geschaltet sein, daß sie unmittelbar nach einem Öffnen und Wiederverschließen des Behälters die Strahlenquelle einschaltet.

Die Zeitschaltelektronik kann darüber hinaus einstellbar sein, so daß gewünschte kürzere und längere Intervalle erreicht werden können.

Zur Überwachung der Strahlenquelle, der Zeitschaltelektronik bzw. der Sicherheitsschaltung können Kontrolleinrichtungen vorgesehen sein, die beim Ausfall oder einer Funktionsstörung den Defekt optisch oder akustisch anzeigen.

Bei einer weiteren Ausführung der Erfindung kann der Behälter über Heiz- oder Kühlvorrichtungen verfügen, so daß auch die gewünschte Temperatur der eingelagerten Lebensmittel eingestellt werden kann, oder beispielsweise mit Miktrowellen, ein Garen der Lebensmittel möglich ist.

Um zu verhindern, daß die eingelagerten Lebensmittel durch Feuchtigkeitsverlust unansehnlich werden, können diese mit einer Folie umhüllt werden, die aus einem UV-lichtdurchlässigen Kunststoff, insbesondere aus einer Fluorpolymerfolie bestehen. Derartige Fluorpolymere sind beständig gegen die UV-Strahlung und daher in der erfindungsgemäßen Vorrichtung vorzugsweise einzusetzen.

Die Erfindung ist in der Zeichnung beispielsweise veranschaulicht und wird im nachfolgenden anhand der Zeichnung im einzelnen beschrieben.

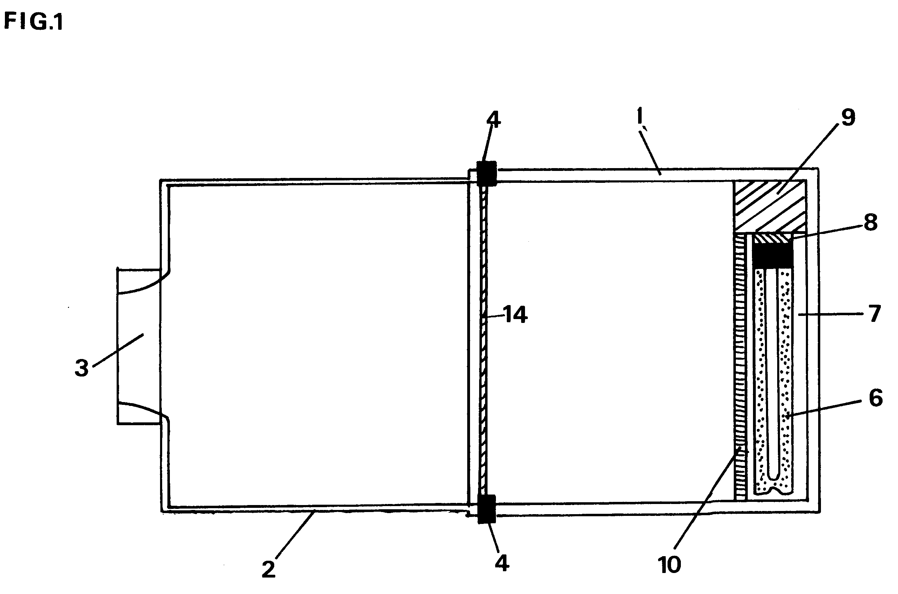

Es zeigen
- Figur 1: eine Draufsicht auf einen Entkeimungsbehälter und

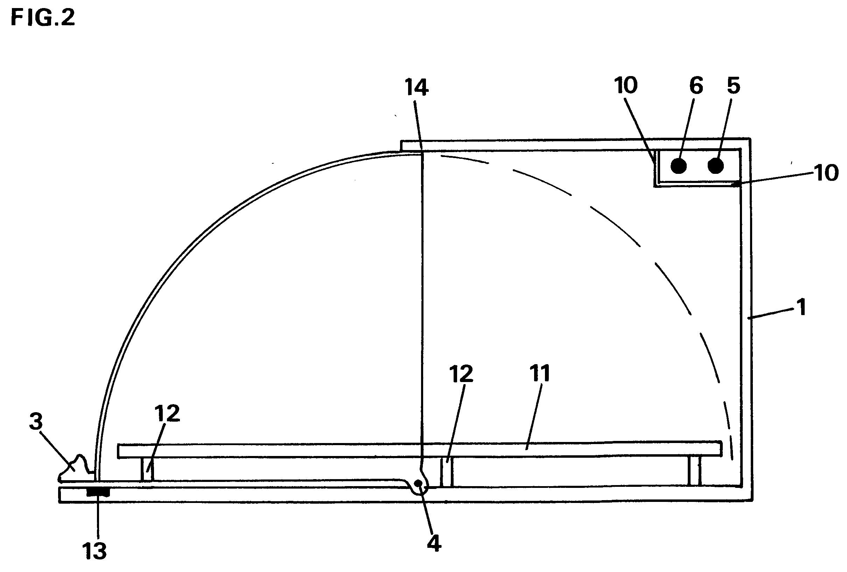

- Figur 2: einen seitlichen Schnitt durch den Entkeimungsbehälter.

An einem hochglanzverspiegelten Gehäuse 1 ist eine um eine Achse 4 drehbare Verschlußkappe 2 mit einem Griff 3 angebracht, die den Behälter nach vorne lichtdicht abschließt und innen ebenfalls verspiegelt ist. Die Verschlußkappe 2 stellt in Verbindung mit einer Dichtleiste 14 sicher, daß bei geschlossenem Behälter kein UV-C-Licht nach außen treten kann.

Im Inneren des Behälters ist eine UV-C-Lampe 6 im Lampenraum 5 angebracht, der komplett von UV-durchlässigen Fenstern 10 aus Quarzglas oder einem UV-lichtdurchlässigen Kunststoff umgeben ist.

Die Lampe 6 steckt mit ihrem Sockel 7 in einer passenden Fassung 8, die ihrerseits am Gehäuse 9 angeordnet und mit der entsprechenden Elektronik verbunden ist. Über diese Elektronik erfolgt die Spannungsversorgung der Lampe 6, die Zeitschaltregelung und die Überwachung der Mikroschalter 13, die die Lampe 6 beim Öffnen des Behälters ausschalten.

Die Mikroschalter sind in Reihe geschaltet, so daß auch beim Ausfall eines Schalters ein Ausschalten der Lampe 6 erfolgt.

Bei geöffneter Verschlußklappe 2 sind der Lampenraum 5 und die Fenster 10 geschützt, da die Verschlußklappe 2 vor diese Einrichtungen geschwenkt ist.

Ein in den Behälter eingezogener Boden 11 besteht ebenso wie die Abstandshalter 12 aus UV-durchlässigem Material, so daß die im Behälter aufbewahrten Gegenstände komplett von UV-C-Licht umflutet sind.

Wie bereits erwähnt, wird der Entkeimungsbehälter vorzugsweise zur keimfreien Aufbewahrung von Lebensmitteln verwendet, es ist jedoch selbstverständlich auch möglich, andere Gegenstände wie ärztliche Instrumente keimfrei aufzubewahren oder zu sterilisieren.

### Bezugszeichenliste

- 1: Gehäuse
- 2: Verschlußklappe
- 3: Griff
- 4: Achse
- 5: Lampenraum
- 6: UVC-Lampe
- 7: Sockel
- 8: Fassung
- 9: Gehäuse
- 10: Fenster
- 11: Boden
- 12: Abstandshalter
- 13: Mikroschalter
- 14: Dichtleiste

## Patentansprüche

1. Vorrichtung zur Entkeimung und keimfreien Aufbewahrung von keimgefährdeten Gegenständen wie Lebensmitteln, Medikamenten, medizinischen Geräten und dergleichen, **gekennzeichnet durch** einen lichtdicht verschließbaren Behälter mit einer UV-Licht aussendenden Strahlenquelle (6), wobei der Behälter eine allseitige, die UV-Strahlen reflektierende Innenverspiegelung aufweist.

2. Vorrichtung nach Anspruch 1, **dadurch gekennzeichnet,** daß der Behälter eine UV-lichtdurchlässige, im Abstand zum Behälterboden angeordnete Auflage (11) aufweist.

3. Vorrichtung nach Anspruch 2, **dadurch gekennzeichnet,** daß die Auflage (11) auf UV-durchlässigen Abstandshaltern (12) auf dem Behälterboden aufgeständert ist.

4. Vorrichtung nach einem der Ansprüche 1 bis 3, **dadurch gekennzeichnet,** daß der Behälter eine Sicherheitsschaltung zum Ausschalten der UV-Strahlenquelle (6) beim Öffnen des Behälters aufweist.

5. Vorrichtung nach einem der Ansprüche 1 bis 4, **dadurch gekennzeichnet,** daß die Sicherheitsschaltung mindestens zwei in Reihe geschaltete Mikroschalter (13) umfaßt.

6. Vorrichtung nach einem der Ansprüche 1 bis 5, **dadurch gekennzeichnet,** daß eine Zeitschaltelektronik zur Begrenzung der Leuchtdauer der UV-Strahlenquelle (6) bzw. zum intervallmäßigen Einschalten der Strahlenquelle (6) vorgesehen ist.

7. Vorrichtung nach einem der Ansprüche 1 bis 6, **dadurch gekennzeichnet,** daß eine Kontrolleinrichtung zur Überwachung der UV-Strahlenquelle (6), der Zeitschaltelektronik und/oder der Sicherheitsschaltung vorgesehen ist.

8. Vorrichtung nach einem der Ansprüche 1 bis 7, **dadurch gekennzeichnet,** daß für den Behälter Heiz- oder Kühleinrichtungen vorgesehen sind.

9. Folie zum Abdecken oder Umhüllen von keimgefährdeten Gegenständen zur Verwendung in einer Vorrichtung gemäß einem der Ansprüche 1 bis 8, **dadurch gekennzeichnet,** daß sie aus einem UV-lichtdurchlässigen Kunststoff, insbesondere aus einem Fluorpolymer besteht.
